# FASCICULE DE BREVET EUROPEEN

(11) **EP 2 757 318 B1**
(45) Date de publication et mention de la délivrance du brevet: **19.08.2015**
(21) Numéro de dépôt: 14354002.9
(22) Date de dépôt: 21.01.2014
(51) Int. Cl.: A61F 9/02, F21V 21/084

(54) **Dispositif de réglage d'une sangle de fixation frontale.**
Vorrichtung zur Anpassung eines Kopfbandes.
Apparatus for adjusting a headband.

(30) Priorité: 21.01.2013 FR 1300129
(43) Date de publication de la demande: 23.07.2014
(73) Titulaire: Zedel, 38920 Crolles (FR)
(72) Inventeur: Girault, Eric, F-74000 Annecy (FR); Martin, Grégory, F-38190 Les Adrets (FR)
(74) Mandataire: Talbot, Alexandre

(56) Documents cités:
- EP-A1- 2 481 981
- US-A1- 2004 128 745
- US-A1- 2006 059 608

## Description

### Domaine technique de l'invention

La présente invention concerne un dispositif de réglage pour élément portatif et, plus particulièrement, un dispositif de réglage amélioré pour élément portatif comprenant des éléments filaires et permettant un réglage simple, rapide et efficace.

### État de la technique

Les dispositifs de réglage pour élément portatif sont très largement utilisés notamment dans le domaine sportif. C'est le cas notamment des dispositifs assurant le bon positionnement des lampes frontales, des masques de ski ou de plongée, des lunettes de natation, etc.

Les dispositifs de réglage actuellement commercialisés, notamment ceux pour les masques de ski, se présentent sous la forme d'une unité unique de réglage de la longueur d'une sangle faisant le tour de la tête et assurant le maintien de l'élément portatif. L'unité de réglage comporte un chemin de coulissement permettant le détournement de la sangle et une zone de fixation de ladite sangle. Associée à l'unité de réglage, un anneau permet à la sangle, passant au travers de l'anneau, de réaliser une boucle entre ledit anneau et l'unité de réglage. L'écartement entre l'anneau et l'unité de réglage permet de régler la longueur de la sangle. Ce type de dispositif présente l'inconvénient d'être peu pratique lorsque l'utilisateur souhaite modifier le réglage sans ôter l'élément portatif et ceci tout en continuant son activité. En effet, pour régler ce type de dispositif, l'utilisateur doit tenir le brin coulissant dans l'unité unique de réglage et faire coulisser sur celui-ci l'unité de réglage. L'utilisateur doit donc d'abord déterminer lequel des deux éléments présents sur la sangle est l'unité de réglage puis lequel des brins est coulissant sur l'unité de réglage. Il doit ensuite effectuer le coulissement de l'unité de réglage le long dudit brin coulissant. Un tel réglage est donc relativement long et laborieux lorsque l'utilisateur est en activité et ceci d'autant que l'unité de réglage et l'anneau sont généralement positionnés à l'arrière de la tête.

Il existe également, pour les éléments portatifs du type lunettes de natation ou de plongée, des dispositifs de réglage se présentant sous la forme de deux unités de réglage disposées spécifiquement de part et d'autre de l'élément portatif, sur le support même dudit élément. Ces types de dispositif sont parfois munis d'une sangle de tour de tête particulière, généralement à base de silicone, comportant un dédoublement fixe au niveau de la partie occipitale de la tête. Une telle sangle permet un bon maintien de l'élément portatif mais le réglage de la longueur de sangle d'un tel dispositif reste difficile à effectuer. En particulier, l'utilisateur à tendance à tirer sur la sangle de réglage plus fortement d'un côté que de l'autre, ceci induisant un réglage non symétrique du dédoublement par rapport au plan sagittal. Le dédoublement, mal positionné, perd alors son efficacité en termes de maintien de l'élément portatif.

Par ailleurs, les dispositifs de l'art antérieur comprennent généralement des sangles spécifiques de type bandeau tissé élastique ou sangle en silicone qu'il est difficile de remplacer lorsqu'elles sont détériorées.

Le document US 2004/0128745 décrit un dispositif de réglage comportant deux cordons coopérant avec deux bloqueurs de cordons. Les bloqueurs de cordons comprennent deux chemins de coulissement pour l'un des cordons et deux organes de blocage mécanique pour les extrémités de l'autre cordon. Le réglage de la longueur du dispositif est effectué en débloquant les bloqueurs de cordon par un double appui sur deux des côtés opposés des bloqueurs et en rapprochant ou en éloignant les deux bloqueurs l'un par rapport à l'autre.

### Objet de l'invention

La présente invention tend à résoudre les inconvénients des dispositifs de réglage traditionnels en proposant un perfectionnement pour les dispositifs de réglage pour élément portatif.

Selon l'invention, ce but est atteint grâce à un dispositif de réglage pour élément portatif comme présenté dans les revendications suivantes et comprenant:
- une première et une deuxième paires de premier et deuxième éléments filaires ;
- une première unité de réglage montée mobile sur la première paire d'éléments filaires et configurée pour :
   ∘ définir deux chemins de détournement de la première paire d'éléments filaires et séparer le premier et le deuxième éléments filaires de la première paire d'une première distance X1,
   ∘ définir un premier organe de couplage mécanique avec la deuxième paire d'éléments filaires ;
- une deuxième unité de réglage montée mobile sur la deuxième paire d'éléments filaires et configurée pour :
   ∘ définir deux chemins de détournement de la deuxième paire d'éléments filaires et séparer le premier et le deuxième éléments filaires de la deuxième paire d'une deuxième distance X2,
   ∘ définir un premier organe de couplage mécanique avec la première paire d'éléments filaires.

### Description sommaire des dessins

D'autres avantages et caractéristiques ressortiront plus clairement de la description qui va suivre de modes particuliers de réalisation de l'invention donnés à titre d'exemples non limitatifs et représentés aux dessins annexés, dans lesquels :

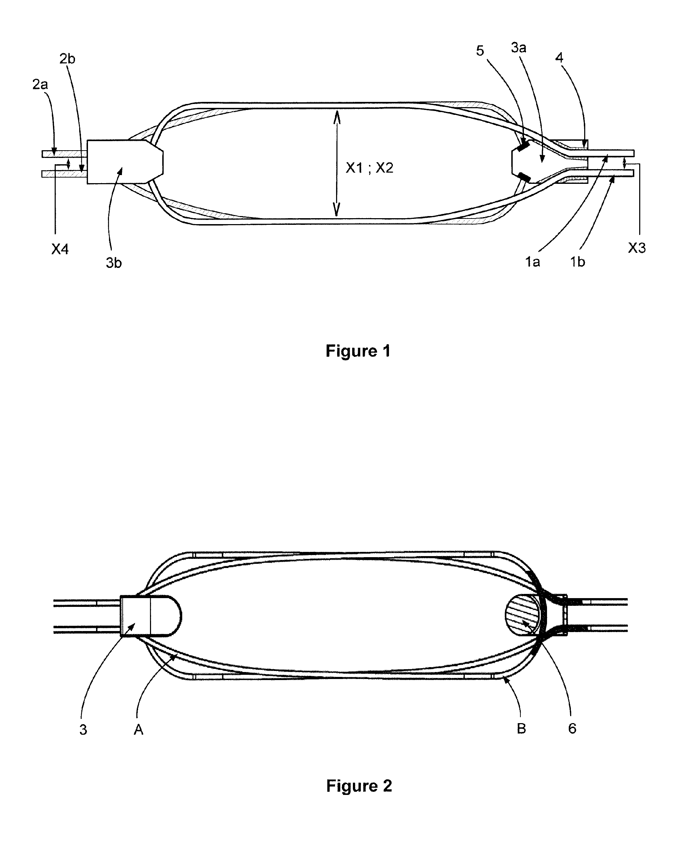

- la figure 1 illustre schématiquement une vue d'un mode de réalisation du dispositif, ladite vue présentant notamment une des faces principales de l'une des unités de réglage (gauche) et une coupe longitudinale parallèle au plan des faces principales de l'autre des unités de réglage (droite) ;
- la figure 2 illustre schématiquement une vue d'un autre mode de réalisation du dispositif, ladite vue présentant notamment une des faces principales de l'une des unités de réglage (gauche) et une coupe longitudinale parallèle au plan des faces principales de l'autre des unités de réglage (droite) ;

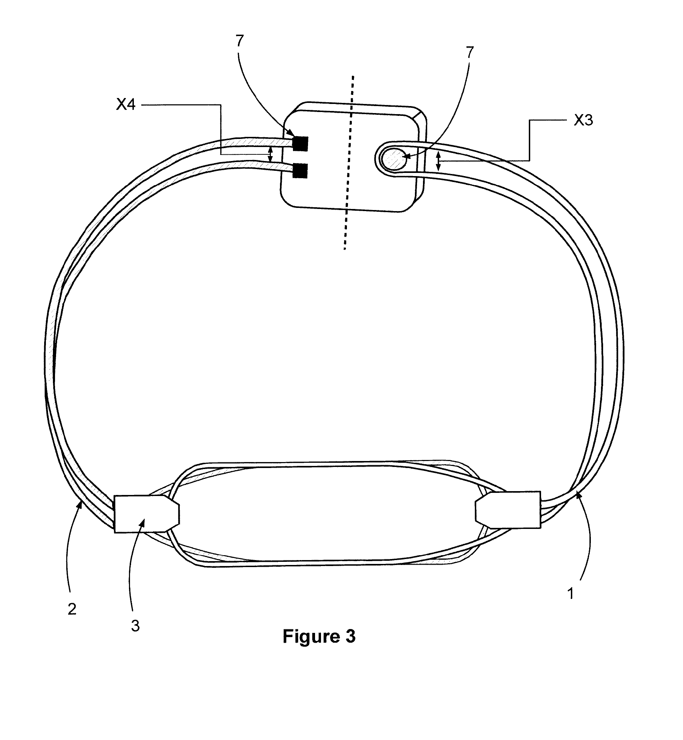

- la figure 3 illustre schématiquement une vue d'un mode de réalisation du dispositif, ladite vue présentant notamment une coupe longitudinale parallèle au plan des faces principales de l'élément portatif et illustrant un mode de réalisation du deuxième organe de couplage mécanique 7 (gauche) et une coupe longitudinale parallèle au plan des faces principales de l'élément portatif et illustrant un autre mode de réalisation du deuxième organe de couplage mécanique 7 (droite).

### Description de modes particuliers de réalisation

Comme illustré schématiquement à la figure 1, le dispositif de réglage pour élément portatif comprend une première et une deuxième paires, respectivement 1 et 2, de premier et deuxième éléments filaires (respectivement 1 a et 1 b, 2a et 2b).

On entend par « élément filaire » tout élément souple longitudinal de longueur très supérieure à son diamètre ou à sa largueur. Il peut s'agir par exemple d'un cordon de diamètre quelconque. Préférentiellement, l'élément filaire est configuré pour présenter une certaine élasticité. De tels éléments filaires présentent l'avantage d'être facilement fixables et remplaçables.

Le dispositif de réglage comprend en outre deux unités de réglage 3, la première unité de réglage 3a étant montée mobile sur la première paire d'éléments filaires 1 et configurée pour :
- définir deux chemins de détournement 4 de la première paire d'éléments filaires 1 et séparer le premier et le deuxième éléments filaires de la première paire 1 d'une première distance X1,
- définir un premier organe de couplage mécanique 5 avec la deuxième paire d'éléments filaires 2,
et la deuxième unité de réglage 3b étant montée mobile sur la deuxième paire d'éléments filaires 2 et configurée pour :
- définir deux chemins de détournement 4 de la deuxième paire d'éléments filaires 2 et séparer le premier et le deuxième éléments filaires de la deuxième paire 2 d'une deuxième distance X2,
- définir un premier organe de couplage mécanique 5 avec la première paire d'éléments filaires 1.

Un tel dispositif permet un réglage rapide, réalisable alors même que l'utilisateur est équipé de l'élément portatif et est, par exemple, en train de pratiquer une activité physique. Il suffit à l'utilisateur de rapprocher entre elles les unités de réglage ou, au contraire, de les éloigner pour obtenir un réglage optimum de la longueur du dispositif.

On entend par «chemin de détournement » tout moyen permettant de faire passer l'élément filaire d'un premier plan vers un deuxième plan, les deux plans pouvant être décalés horizontalement, verticalement et/ou latéralement. Le chemin de détournement est configuré pour empêcher le coulissement non contrôlé de l'unité de réglage 3 le long de l'élément filaire dès lors que l'élément filaire est en tension de part et d'autre de l'unité de réglage 3. Le chemin de détournement induit une modification de la trajectoire de l'élément filaire. Ainsi, lorsqu'une tension est appliquée sur l'élément filaire uniquement d'un côté de l'unité de réglage 3, l'élément filaire coulisse dans le chemin de détournement 4. Lorsqu'une même tension est appliquée sur l'élément filaire de part et d'autre de l'unité de réglage 3, l'élément filaire reste fixe et ne coulisse plus dans le chemin de détournement 4. Cette fonction est d'autant plus assurée qu'il existe deux chemins de détournement par unité de réglage. Chaque élément filaire passe dans un chemin de détournement dédié.

La première distance X1 entre le premier et le deuxième éléments filaires de la première paire 1 et la deuxième distance X2 entre le premier et le deuxième éléments filaires de la deuxième paire 2 représentent les distances d'écartement des premier et deuxième éléments filaires entre les unités de réglage 3. Un tel écartement des éléments filaires entre les unités de réglage assure ainsi un excellent maintien de l'élément portatif grâce notamment à un double appui des éléments filaires écartés. Selon un mode de réalisation préféré, cet écartement est situé à l'opposé de l'élément portatif. A titre d'exemple, lorsque l'élément portatif est porté sur le front ou le visage de l'individu, l'écartement est alors positionné à l'arrière de la tête. Le double appui induit par l'écartement est avantageusement tel que chaque élément filaire d'une même paire se positionne symétriquement de part et d'autre du plan passant par l'élément portatif, ledit plan étant perpendiculaire au plan sagittal lorsque l'élément portatif est placé sur le visage ou le front de l'individu. Avantageusement, les distances X1 et X2 sont identiques.

Selon un mode de réalisation préféré, les chemins de détournement 4 d'une unité de réglage 3 sont configurés pour séparer le premier et le deuxième éléments filaires d'une même paire d'une distance X (respectivement X3 pour la première paire 1 et X4 pour la deuxième paire 2). Les distances de séparation X3 et X4 correspondent à l'écartement des éléments filaires d'une même paire existant entre les éléments filaires reliant une unité de réglage à l'élément portatif. Une telle séparation des éléments filaires permet un meilleur coulissement des éléments filaires dans les chemins de détournement dès lors que lesdits éléments filaires ne sont pas en tension simultanément d'un côté et de l'autre du chemin de détournement 4. Avantageusement, les distances X3 et X4 sont identiques.

A titre d'exemple et comme illustré aux figures 1 et 2, le chemin de détournement peut consister en un trou traversant compris dans l'épaisseur de l'unité de réglage, ledit trou reliant une première face latérale de l'unité de réglage à une deuxième face latérale adjacente de cette même unité de réglage. Selon un mode de réalisation avantageux, notamment lorsque l'élément portatif est une lampe frontale, l'une des faces principales de l'unité de réglage s'entend de la face en contact avec la tête ou un casque, l'autre des faces principales lui étant parallèle et positionnée dans un plan postérieur. Les faces latérales de l'unité de réglage s'entendent des faces reliant entre elles les deux faces principales.

Chaque unité de réglage 3 comprend également un organe de couplage mécanique 5. Le couplage entre un élément filaire et une unité de réglage 3 peut s'effectuer par tout moyen bien connu de l'homme du métier, par exemple par des techniques de nouage, de couture, de soudage ou de collage. Le couplage grâce à un ou plusieurs noeuds présente l'avantage de pouvoir remplacer facilement les éléments filaires dès lors qu'ils sont endommagés. On peut avoir un couplage mécanique fixe ou un couplage mécanique permettant à chaque unité de réglage d'être montée mobile sur une paire d'éléments filaires.

Les unités de réglage 3 peuvent être de tout type et de toute forme appropriée. En particulier, elles peuvent être en matière plastique ou en métal. Préférentiellement, les unités de réglage présentent deux faces principales telles que définies précédemment et au moins une face latérale reliant entre elles lesdites faces principales. Les deux unités de réglage sont avantageusement identiques.

Selon un mode de réalisation préféré, l'élément portatif est configuré pour :
- définir un deuxième organe de couplage mécanique 7 avec la première paire d'éléments filaires 1 et séparer le premier et le deuxième éléments filaires de la première paire 1 d'une troisième distance X3,
- définir un deuxième organe de couplage mécanique 7 avec la deuxième paire d'éléments filaires 2 et séparer le premier et le deuxième éléments filaires de la deuxième paire 2 d'une quatrième distance X4.
Une telle séparation des éléments filaires permet un meilleur coulissement des éléments filaires dans les chemins de détournement dès lors que lesdits éléments filaires ne sont pas en tension simultanément d'un côté et de l'autre du chemin de détournement 4.

Avantageusement, ce dernier mode de réalisation est associé avec le mode de réalisation précédemment décrit dans lequel les chemins de détournement 4 d'une unité de réglage 3 sont configurés pour séparer le premier et le deuxième élément filaire d'une même paire d'une distance X (respectivement X3 pour la première paire 1 et X4 pour la deuxième paire 2). Avantageusement, les distances X3 et X4 sont identiques.

Selon un mode de réalisation préféré du dispositif et comme illustré à la figure 2, la première et la seconde paires d'éléments filaires, respectivement 1 et 2, forment chacune un élément filaire unique, respectivement A et B, et les organes de couplage mécanique 5 de chaque unité de réglage 3 forment un organe de renvoi 6 pour un des éléments filaires A ou B. L'élément filaire A ou B est ainsi monté coulissant sur chacune des unités de réglage. Avantageusement, l'organe de renvoi est configuré pour augmenter la distance de séparation X3, X4 par rapport aux distances de séparation X1 et X2. Au moyen de l'organe de renvoi, l'élément filaire se déplace avantageusement suivant une direction perpendiculaire à la direction définie par l'élément filaire entre l'élément portatif et l'unité de réglage.

Selon un mode de réalisation préféré, l'organe de renvoi 6 est configuré pour permettre à l'élément filaire A ou B de faire une boucle dont la largeur est sensiblement égale à la distance X3 pour l'élément filaire A et X4 pour l'élément filaire B.

Selon un mode de réalisation particulièrement préféré du dispositif, les éléments filaires sont de même longueur. Un tel dispositif permet ainsi un réglage symétrique de l'élément portatif. La tension exercée par les deux paires d'éléments filaires de part et d'autre de l'élément portatif est alors toujours de même intensité, garantissant un confort optimum pour l'utilisateur. Un positionnement symétrique des unités de réglage 3 par rapport au plan vertical passant par l'élément portatif permet également le maintien en place de l'élément portatif alors même que l'utilisateur est en train de régler la longueur du dispositif. Avantageusement, ledit plan vertical passant par l'élément portatif correspond au plan sagittal lorsque l'élément portatif est positionné sur le visage ou le front.

Selon un mode de réalisation préféré, les éléments filaires forment une boucle continue. L'élément portatif peut alors être monté coulissant sur ladite boucle continue.

Selon un mode de réalisation avantageux, l'élément portatif est une lampe frontale.

L'élément portatif peut être configuré pour être placé sur la tête, un casque, un bras ou un autre élément. Préférentiellement, le dispositif de réglage associé à l'élément portatif est configuré pour être placé sur la tête de l'utilisateur ou sur un casque.

En comparaison d'une fixation par sangle, les éléments filaires permettent une plus grande facilité d'utilisation car la surface entre les éléments filaires peut varier facilement.

## Revendications

1. Dispositif de réglage pour élément portatif comprenant :
- une première et une deuxième paires de premier et deuxième éléments filaires (1 ; 2) ;
- une première unité de réglage (3a) montée mobile sur la première paire d'éléments filaires (1) et configurée pour :
∘ définir un premier organe de couplage mécanique (5) avec la deuxième paire d'éléments filaires (2) ;
- une deuxième unité de réglage (3b) montée mobile sur la deuxième paire d'éléments filaires (2) et configurée pour :
∘ définir un premier organe de couplage mécanique (5) avec la première paire d'éléments filaires (1).
Dispositif de réglage **caractérisé en ce que**
- la première unité de réglage (3a) est configurée pour définir deux chemins de détournement (4) de la première paire d'éléments filaires (1) et séparer le premier et le deuxième éléments filaires de la première paire (1) d'une première distance (X1),
- la deuxième unité de réglage (3b) est configurée pour définir deux chemins de détournement (4) de la deuxième paire d'éléments filaires (2) et séparer le premier et le deuxième éléments filaires de la deuxième paire (2) d'une deuxième distance (X2).

2. Dispositif de réglage pour élément portatif selon la revendication 1, **caractérisé en ce que** l'élément portatif est configuré pour :
- définir un deuxième organe de couplage mécanique (7) avec la première paire d'éléments filaires (1) et séparer le premier et le deuxième éléments filaires de la première paire (1) d'une troisième distance (X3),
- définir un deuxième organe de couplage mécanique (7) avec la deuxième paire d'éléments filaires (2) et séparer le premier et le deuxième éléments filaires de la deuxième paire (2) d'une quatrième distance (X4).

3. Dispositif de réglage pour élément portatif selon l'une ou l'autre des revendications 1 et 2, **caractérisé en ce que** :
- la première et la seconde paires d'éléments filaires (1 ; 2) forment chacune un élément filaire unique (A ; B), et
- les organes de couplage mécanique (5) de chaque unité de réglage (3) forment un organe de renvoi (6) pour un des éléments filaires (A ; B).

4. Dispositif de réglage pour élément portatif selon l'une quelconque des revendications précédentes, **caractérisé en ce que** les éléments filaires sont de même longueur.

5. Dispositif de réglage pour élément portatif selon l'une quelconque des revendications précédentes, **caractérisé en ce que** les éléments filaires forment une boucle continue.

6. Dispositif de réglage pour élément portatif selon l'une quelconque des revendications précédentes, **caractérisé en ce qu'**il permet la fixation d'une lampe frontale.

## Patentansprüche

1. Verstellvorrichtung für ein tragbares Element, die umfasst:
- ein erstes und ein zweites Paar erster und zweiter Drahtelemente (1; 2);
- eine erste Verstelleinheit (3a), die beweglich an das erste Paar Drahtelemente (1) montiert und so ausgebildet ist, dass:
∘ sie eine erste mechanische Vorrichtung (5) zur Verbindung mit dem zweiten Paar Drahtelemente (2) bildet;
- eine zweite Verstelleinheit (3b), die beweglich an das zweite Paar Drahtelemente (2) montiert und so ausgebildet ist, dass:
∘ sie eine zweite mechanische Vorrichtung (5) zur Verbindung mit dem ersten Paar Drahtelemente (1) bildet.
verstellvorrichtung **dadurch gekennzeichnet dass**
- die erste Verstelleinheit (3a) so vorgesehen ist, dass sie zwei Umkehrwege (4) des ersten Paars Drahtelemente (1) bildet und das erste und zweite Drahtelement des ersten Paars (1) durcht einen ersten Abstand (X1) voneinander trennt,
- die zweite Verstelleinheit (3b) so vorgesehen ist, dass sie zwei Umkehrwege (4) des zweiten Paars Drahtelemente (2) bildet und das erste und zweite Drahtelement des zweiten Paars (2) durch einen zweiten Abstand (X2) voneinander trennt.

2. Verstellvorrichtung für ein tragbares Element nach Anspruch 1, **dadurch gekennzeichnet, dass** das tragbare Element so ausgestaltet ist, dass es:
- eine zweite mechanische Vorrichtung (7) zur Verbindung mit dem ersten Paar Drahtelemente (1) bildet und das erste und zweite Drahtelement des ersten Paars (1) durch einen Abstand (X3) voneinander trennt,
- eine zweite mechanische Vorrichtung (7) zur Verbindung mit dem zweiten Paar Drahtelemente (2) bildet und das erste und zweite Drahtelement des zweiten Paars (2) durch einen Abstand (X4) voneinander trennt.

3. Verstellvorrichtung für ein tragbares Element nach einem der Ansprüche 1 und 2, **dadurch gekennzeichnet, dass**:
- das erste und zweite Paar Drahtelemente (1; 2) jeweils ein einziges Drahtelement (A; B) bilden, und
- die mechanischen Verbindungsvorrichtungen (5) jeder Verstelleinheit (3) eine Umlenkvorrichtung (6) für eines der Drahtelemente (A; B) bilden.

4. Verstellvorrichtung für ein tragbares Element nach einem der vorstehenden Ansprüche, **dadurch gekennzeichnet, dass** die Drahtelemente die gleiche Länge haben.

5. Verstellrrorrichtung für ein tragbares Element nach einem der vorstehenden Ansprüche, **dadurch gekennzeichnet, dass** die Drahtelemente eine durchgehende Schleife bilden.

6. Verstellvorrichtung für ein tragbares Element nach einem der vorstehenden Ansprüche, **dadurch gekennzeichnet, dass** sie die Befestigung einer Stirnlampe ermöglicht.

## Claims

1. An adjustment device for a portable element comprising:
- a first and a second pair of first and second wire-shaped elements (1; 2);
- a first adjustment unit (3a) mounted mobile on the first pair of wire-shaped elements (1) and configured to:
∘ define a first mechanical coupling means (5) with the second pair of wire-shaped elements (2);
- a second adjustment unit (3b) mounted mobile on the second pair of wire-shaped elements (2) and configured to:
∘ define a first mechanical coupling means (5) with the first pair of wire-shaped elements (1),
Adjustment device **characterized in that**
- the first adjustment unit (3a) is configured to define two deviation paths (4) of the first pair of wire-shaped elements (1) and to separate the first and second wire-shaped elements of the first pair (1) by a first distance (X1),
- the second adjustment unit (3b) is configured to define two deviation paths (4) of the second pair of wire-shaped elements (2) and to separate the first and second wire-shaped elements of the second pair (2) by a second distance (X2).

2. Adjustment device for a portable element according to claim 1, **characterized in that** the portable element is configured to:
- define a second mechanical coupling means (7) with the first pair of wire-shaped elements (1) and to separate the first and second wire-shaped elements of the first pair (1) by a third distance (X3),
- define a second mechanical coupling means (7) with the second pair of wire-shaped elements (2) and to separate the first and second wire-shaped elements of the second pair (2) by a fourth distance (X4).

3. Adjustment device for a portable element according to one of the claim 1 and 2, **characterized in that**:
- the first and second pairs of wire-shaped elements (1; 2) each form a single wire-shaped element (A; B), and
- the mechanical coupling means (5) of each adjustment unit (3) form a return means (6) for one of the wire-shaped elements (A; B).

4. Adjustment device for a portable element according to anyone of the preceding claims, **characterized in that** the wire-shaped elements are of the same length.

5. Adjustment device for a portable element according to anyone of the preceding claims, **characterized in that** the wire-shaped elements form a continuous loop.

6. Adjustment device for a portable element according to anyone of the preceding claims, **characterized in that** it enables fixing of a headlamp,
